# EUROPEAN PATENT SPECIFICATION

(11) **EP 0 643 297 B1**
(45) Date of publication and mention of the grant of the patent: **18.12.2002**
(21) Application number: 94202609.7
(22) Date of filing: 09.09.1994
(51) Int. Cl.: G01N 25/72, G01N 33/38

(54) **Analytical system for analyzing, monitoring, diagnosing and/or controlling a process for manufacturing packaging glass products in which the analysis takes place directly after the glass-shaping process**
Analytisches System zum Analysieren, Überwachen, Diagnostizieren und/oder Steuern eines Verfahrens für die Herstellung von Verpackungsprodukten aus Glas, woin die Analyse sofort nach dem Glasformungsverfahren stattfindet
Système analytique pour analyser, contrôler, diagnostiquer et/ou réguler un procédé pour la fabrication de produits d'emballage en verre, dans laquelle l'analyse à lien directement après le procédé du formage du verre

(30) Priority: 09.09.1993 NL 9301568
(43) Date of publication of application: 15.03.1995
(73) Proprietor: TCE CONSULTANCY & ENGINEERING, NL-9951 JZ Winsum (NL)
(72) Inventor: Troost, Dirk Johannes, NL-9951 JZ Winsum (NL)
(74) Representative: Smulders, Theodorus A.H.J., Ir.

(56) References cited:
- EP-A- 0 153 565
- EP-A- 0 177 004
- WO-A-93/11410
- US-A- 5 032 727
- IEEE TRANSACTIONS ON PATTERN ANALYSIS AND MACHINE INTELLIGENCE, vol.PAMI-5, no.6, November 1983, NEW YORK US pages 563 - 572 B.R. SURESH, ET AL. 'A Real-Time Automated Visual Inspection System for Hot Steel Slabs'

## Description

The invention relates to an analytical system for analyzing, monitoring, diagnosing and/or controlling a process for manufacturing packaging glass products.

The invention also relates to an apparatus for manufacturing, partly by heating, packaging glass products provided with an analytical system in which a glass material to be shaped into the packaging glass product is passed along a production stage comprising a first section in which the glass material is molten, a second section, following the first section, in which the product is shaped from the glass material, and a third section, following the second section, in which the glass material shaped into a product is cooled, the infrared-sensitive sensor system detecting products at at least a point in the second section in which the shaped products are not yet cooled.

Such analytical systems are known from EP-0 177004.

A problem in these processes is that the waste of products is relatively high. This is partly caused by the fact that the products are inspected after they have passed through the critical part of the production process. In a glass blowing machine in which blown products traverse a stage through an annealing furnace this means that the products are inspected by means of a video camera when leaving the annealing furnace. In EP-177004 the glass product is illuminated with a light source for inspection by means of a measuring unit comprising photodiodes.

If at this point of the production process it is found that the quality of the product is not sufficient and/or the product contains deviations, there is a great chance that all the other products which have meanwhile been shaped after the inspected product or products and, e.g., are still present in the annealing furnace will have the same or a comparable problem and must be rejected accordingly. In other words, at the moment it is found in a productions process that products do not meet a specific quality standard or show deviations large amounts of other products with the same problems have meanwhile been manufactured. When the production process is then readjusted, optimized, yet a large amount of products will have to be rejected.

Deviations which may occur in the product can be caused by, e.g., contamination of the material from which the product is made. This contamination may already be present in the base material. before this is shaped into a product. It is also possible, however, that the material is contaminated during shaping of the product in the production process.

The quality of the product may be connected with, e.g., the thickness of the material when it has been shaped into a product. In case of bottles one may think of the wall thickness of the bottle, while in case of enamelling one may think of, e.g., the thickness of the enamel layer.

Besides, the known analytical systems have the drawback that the quality of a product and/or deviations in a product often cannot be measured accurately.
The invention meets the above drawbacks and is characterized in that said analytical system is provided with an infrared-sensitive sensor system and a digital processor connected therewith, said infrared-sensitive sensor system detecting infrared radiation emitted by warm products in the section directly after the glass-shaping process, and said digital processor determining the energy distribution in the material of the shaped product and energy differences between different parts of the shaped product by means of information from the products obtained with said infrared-sensitive sensor system wherein said energy distribution and/or energy differences are compared with criteria, obtained by means of a mathematical reference model, for determining deviations in glass distributions and causes leading to thermal stresses in the product.

The emitted radiation of a semitransparent material depends on the local glass thickness as well as on the internal (thermal) energy of the glass obtaining during the glass-shaping process. Thus, e.g., a thicker part of the glass will contain and emit more energy than a thinner part of the glass. By detecting the emitted radiation, i.e. by detecting the energy distribution in the material of the shaped product and/or energy differences between different parts of the shaped product information may therefore be obtained about the local glass thickness as well as the internal (thermal) stresses of the glass emitting the relevant radiation. By detecting the emitted radiation the glass distribution of the glass and/or deviations of the glass can be determined by means of a mathematical model. Likewise, it is possible to determine the thermal energy and to compare it with a mathematical model. Thus, packaging glass products with an inadmissable thermal stress can be selected. Thermal stresses in the glass are caused during the glass-shaping process by the occurrence of unduly high (thermal) energy differences during the glass-shaping process. The above mathematical model has been developed by means of specific physical properties, such as the released (emitted) infrared radiation in combination with specific sizes and glass composition of the product. The invention is therefore based on the fundamental idea that the emitted radiation of the packaging glass product not yet cooled can be directly connected with the energy distribution in the material of the shaped product and/or the energy differences between different parts of the shaped product. Subsequently, from the above thermal energy distribution and/or thermal energy differences the glass distribution over the product and/or thermal stresses in the product can be determined.

Because the analytical system according to the invention analyzes products that are still warm, earlier intervention, seen in the order of time, in a production process is possible, if the product does not meet specific requirements, without the necessity of rejecting large amounts of earlier shaped products. Besides, it turns out that by means of the above energy differences and/or energy distribution products can be analyzed much more accurately in comparison with the known analytical systems.

According to the invention the above energy differences and/or energy distribution are compared with criteria, obtained by means of a mathematical reference model, for determining deviations in glass distribution and/or causes leading to thermal stresses in the product. A deviation in a product of glass will be caused by, e.g., an undesirable glass distribution. These deviations will result in energy differences in the warm glass detected by the analytical system by means of the radiation received.

In particular, the above criteria depend on the associated location on or within the material of the product for which the energy differences and/or energy distribution are determined. Thus, critical parts of a product, such as the neck of a bottle, can be tested, if desired, for criteria more severe than those for other less critical parts of the product.

According to another aspect of the invention the sensor system comprises at least a detector element with infrared-sensitive pixels arranged in rows or rows and columns. In particular, the digital processor determines differences between the energy content of different pixels for obtaining the above energy differences between different parts of the product.

According to a more advanced embodiment of the invention the digital processor comprises a cluster-former for localizing clusters of adjacent pixels comprising at least substantially the same energy content, which digital processor determines energy differences between localized clusters for obtaining the above energy differences between different parts of the product.

According to a further special embodiment the energy differences are determined with coordinates reproducing the associated pixels of the detector element. Here, according to another aspect of the analytical system, in use, the above coordinates will correspond with coordinates representing a part of the product. Thus,he quality of and deviations in products can be determined as a function of their location on or in the product. For this purpose, the detector element preferably comprises an infrared-sensitive sensor.

According to a special embodiment of the invention the digital processor determines the glass distribution of the product by means of the energy distribution over the product. By means of the glass distribution, e.g., the wall thicknesses of a shaped bottle can be determined. These wall thicknesses are then employed as a measure of the quality of the bottle.

In particular, the digital processor determines a three-dimensional glass distribution of the product. For this purpose, the sensor system may be provided with a number of detector units detecting the product from different positions in space.

According to another aspect of the invention the digital processor compares the glass distribution with criteria obtained with the mathematical model for obtaining the information about deviations in the product. In particular, the digital processor determines the causes leading to thermal stresses in the product by means of the energy differences and compares the with the developed reference model for obtaining parameters indicating a deviation in the product.

According to a special embodiment of the invention the infrared-sensitive sensor system, in use, detects products having a temperature above 100°C. More in particular, the infrared-sensitive sensor system, in use, detects products having a temperature above 400°C.

According to another aspect of the invention, in use, process parameters of the production process are regulated on the basis of the parameters about deviations in and/or quality of a product or series of products. More in particular, the above control signals are generated when the parameters obtained trendwise reproduce defects or a deviation of the desired quality.

In an apparatus according to the invention in which glass products are produced, partly by heating, which apparatus is provided with an analytical system as described above, a glass material to be shaped into the product is passed along a production stage comprising, a first section in which the glass material is heated, a second section, following the first section, in which the product is shaped from the glass material, and a third section, following the second section, in which the glass material shaped into a product is cooled, the infrared-sensitive sensor system detecting products at a point in the second section directly after the glass-shaping process.

WO/93/11410 discloses a method for analyzing non hollow glass drips during the manufacturing process of the drips. However , the method is not suitable to be used for hollow glass packaging products with a varying local glass thickness.

The invention will be explained in more detail with reference to the figure which shows an embodiment of an analytical system according to the invention.

In the figure reference numeral 1 denotes an analytical system according to the invention. Reference numeral 2 denotes an apparatus according to the invention for producing packaging glass products 4 which are subjected to a heat treatment during the production process, and which apparatus 2 also comprises the analytical system 1.

Before describing the analytical system 1, there will first be discussed a possible production process of the apparatus 2 in which the analytical system 1 can be used successfully.

In this example the apparatus 2 comprises a schematically shown production line 6 in which the product 4 or the products 4 traverse(s) a production stage in the direction of the arrow 8. In this example the production line 6 comprises a feed unit 10 which delivers heated material 12 from which a product 4 can be manufactured. The heated material 12 is fed to a product-shaping unit 14, if required together with other materials and/or semimanufactures, not further specified. The product-shaping unit 14 manufactures, at least partly from the hot material 12, a product 4 which is still hot immediately after shaping. The manufactured product moves in the direction of the arrow 8 while gradually cooling. For this purpose, the apparatus may be provided with, e.g., an annealing furnace 16, not further specified.

In this example the production process comprises a first section 18 in which the material 12 is heated, a second section 20 in which the material is shaped into a product 4, and a third section 22 in which the product is cooled.

The above production line 6 may produce, e.g., glass bottles 4. The feed unit 10 comprises known means for producing a portion of molten glass. The product-shaping unit 14 comprises molds known per se in which a portion of heated glass 12 is deposited and blowing means for blowing or pressing the glass in the mold to obtain the final shape. The bottles thus formed are then fed to the annealing furnace 16. In known apparatuses the bottles thus obtained and leaving the annealing furnace (cold-end inspection) are inspected for defects (e.g., contamination or air bubbles in the glass) by means of a video camera. When a deviation has been found, the cause thereof can be examined, and the production process can be adapted accordingly to remove the deviation. It is a great disadvantage that all the products already produced and, e.g., still being in the annealing furnace 16 often comprise the same deviation and must therefore be rejected. Besides, it turns out that by no means all of the relevant deviations can be detected with a video camera. In order to solve these problems, the apparatus is provided with the analytical system 1.

According to the invention the analytical system 1 is provided with an infrared-sensitive sensor system 24 comprising at least a infrared-sensitive detector element 26. In this case the detector element 26 comprises an infrared-sensitive sensor of a generally known type. The pixels of the detector element 26 are arranged in vertical and/or vertical and horizontal rows. Each pixel generates a signal representing the energy content of the infrared radiation detected by that pixel. In use, this will be, as in the present practical example, in particular the radiation of a product just manufactured in a production process, at least partly from a heated material, and not yet cooled.

The signals generated by the sensor system 24 are transmitted via line 28 to a digital processor 30 which analyzes the signals and generates parameters representing the results of this analysis. Besides, if necessary, control signals S are generated to adapt process parameters of the production line 6 and/or, on the other hand, to intervene in the production line 6, e.g. by automatically replacing defective component parts.

The sensor system is preferably so positioned that, seen in the direction of the arrow 8, products located in a first part of the third section 22 or before the third section 22 are detected. Possible positions of the sensor system 24 are indicated by the arrow 31. Preferably, when detected by the sensor system, the products shaped will have a temperature above 100°C.

In this example the infrared-sensitive sensor system 24 is so positioned that products are detected immediately after leaving the product-shaping unit 14 and before entering the annealing furnace 16.

According to a possible embodiment of the digital processor 30 it comprises an interface 32 sampling and digitalizing the signals of line 28. These digitalized signals are transmitted to an energy difference measuring unit 36 directly or, if required, via a unit 34 to be described further. By means of the digitalized values of signals of the pixels representing the energy content, the energy difference measuring unit 36 determines energy differences between and belonging to these pixels. The energy differences thus obtained, more in particular signals representing these energy differences or magnitudes corresponding therewith, are transmitted to an analytical unit 38. When the products contain a deviation, such as inhomogeneity and/or causes leading to thermal stresses in the glass, this will be expressed in a deviation of the energy differences to be normally expected in the product 4. The lastmentioned signals therefore contain information about possible deviations in a product.

The analytical unit 38 compares these signals with criteria values determined according to a mathematical model, stored in a criteria unit 40, and transmitted to the analytical unit 38 via line 42. These criteria can be adjusted to the specific production process in which the analytical system is used.

when, e.g., at a specific point of a product the energy difference exceeds a specific value, a parameter indicating that the product contains a deviation at that place can be generated on line 43. More in particular, such a parameter is generated when it turns out that a number of products contain such an energy difference successively at the same place. The criteria may be different for different parts of the product. For this reason the coordinates of pixels for which the energy difference measuring unit 36 has determined energy differences are preferably also transmitted to the analytical unit 38. Thus, the coordinates of possible parts of products for which energy differences have been determined are also known. In other words, these coordinates correspond to coordinates representing a part of the product.

The analytical unit can find out by means of position-dependent criteria whether a specific part of a product contains a deviation.

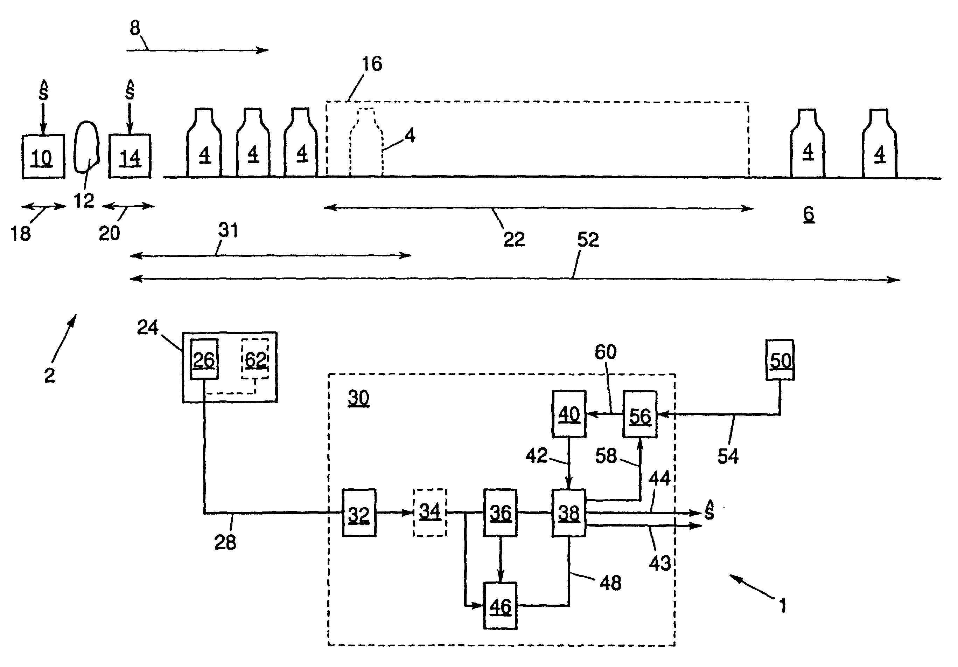

The analytical unit 38 transmits the result of the analysis conducted to line 43 in the form of parameters which represent causes leading to thermal stresses in the product. Moreover, these parameters may contain an indication of the cause of the deviation so that a diagnosis is made. When a deviation has been found, the analytical unit 38 can transmit a control signal S to line 44, if necessary, with which process parameters of the production process of the apparatus 2 are controlled or, on the other hand, intervention in the production process takes place. The arrows shown in Fig. 1 may or may not be combined or extended. In particular, these control signals are generated only if a deviation from the criteria has been found trendwise by the analytical unit 38. In this example the control signals are transmitted to the product-shaping unit and/or the feed unit via line 44 in order to readjust the process parameters of these units. In this example one may think of the pressure at which the glass is blown or the temperature of the mold in which the glass is shaped. Besides, a control signal can be produced to the effect that, e.g., a mold must be replaced if it has been found by the analytical unit 38 that this mold shows a deviation.

The digital processor is further provided with an energy distribution measuring unit 46 which, by means of the signals produced by the unit 32, calculates an energy distribution and from this a glass distribution of a detected product 4. Calculation of such a glass distribution can be realized with means known per se. The resulting glass distribution, more in particular glass distribution signals representing the glass distribution or critical parts of the glass distribution of a product, is transmitted to the analytical unit 38 via line 48. In this case these glass distribution signals contain information about, e.g., the thickness of the glass wall of a bottle, or informatiomn about, e.g., the thickness of a part of the glass wall of the bottle and therefore give an indication of the quality of the bottle. The analytical unit 38 compares these glass distribution signals with criteria stored in the criteria unit 40. When a deviation in the quality of the product 4 is found, more in paricular, when such a deviation is found trendwise, the analytical unit 38 again generates a control signal S to readjust the production process, in this case the product-shaping unit 14 or the feed unit 10, or, on the other hand, to control the production process by, e.g., replacing a mold so that the deviation in the glass distribution will disappear.

Besides, in connection with an examination conducted by means of the criteria, the analytical unit 38 generates parameters which are a measure of the deviation found in the glass distribution, and which are therefore a measure of the quality of the product 4. These parameters are transmitted to line 43 and can be shown, if desired, on a display, not shown.

By the expression trendwise, as used above, is meant a deviation in glass distribution and causes leading to thermal stresses which repeatedly occur or trendwise show a deviation in different products.

The analytical system 1 may further be provided with, e.g., an optical sensor 50 capable of detecting visible light and/or ultraviolet light. The optical sensor 50 detects products 4 which are still warm or have at least partly been cooled. It is also possible, however, that the optical sensor 50 is positioned in a last part of the third section 22. Possible positions of the optical sensor 50 are indicated by the arrow 52.

By means of the optical sensor an image of the product 50 is obtained. This image is transmitted via line 54 to an image-analyzing unit 56 which, by means of known techniques, checks whether the product contains deviations. The parameters generated by the analytical unit 38 for the same products, which parameters show deviations in and/or the glass distribution of the products, are also transmitted to the image analyzing unit 56 via line 58. When it turns out that information obtained by means of the infrared-sensitive sensor system 24 (deviations and/or glass distribution) about the products does not correspond to the information obtained by means of the optical sensor (deviations and/or glass distribution) of the same products, the criteria with which the data provided by the infrared-sensitive sensor system have been analyzed can be adjusted. The adjustment of these criteria must be such that the information determined by means the two sensors 24, 50 is in agreement. The adjustment of the criteria can be carried out by the image analyzing unit via line 60. This involves that the analytical system 1 according to the invention passes through a learning process in which the system gets to know better the production line 6 with the production process. This enables the analytical system 1 to adjust itself to any production process so that process parameters can be regulated successfully.

According to a special embodiment of the digital processor 30 there is further provided a unit 34 which determines clusters of adjacent pixels with at least substantially the same energy content. The information from these clusters , i.e. the energy content and position, is transmitted to the unit 36. It is an advantage that unit 36 determines energy differences of regions that are much larger than regions of a few pixels so that the amount of arithmetical work can be limited.

Finally, it is observed that the infrared-sensitive sensor system 24 may be provided with a second sensor unit 62 positioned at some distance from the first sensor unit, and the signals of which are digitalized with the interface 32 and then transmitted to the cluster-forming unit 34 (if present) or directly to the energy difference measuring unit 36. According to the invention a three-dimensional glass distribution of a product can be determined by the energy distribution measuring unit 46 by detecting a product 4 by the sensor system 24 from two positions spaced from each other.

It is emphatically observed that only a possible embodiment of the digital processor 30 has been described. It is also possible, e.g., that functions of the cluster-forming unit 34, energy distribution measuring unit 36, analytical unit 38, image-analyzing unit 56, criteria unit 42 and/or energy distribution measuring unit 46 are realized by a single digital signal processor, the various functions being defined in the software.

## Claims

1. An analytical system (1) for analyzing, monitoring, diagnosing and/or controlling a process for manufacturing packaging glass products (4) said analytical system (1) being provided with an infrared-sensitive sensor system (24) and a digital processor (30) connected therewith, said infrared-sensitive sensor system (24) detecting infrared radiation emitted by warm products in the section directly after the glass-shaping process, and said digital processor (30) determining the energy distribution in the material of the shaped product and energy differences between different parts of the shaped product by means of information from the products obtained with said infrared-sensitive sensor system wherein said energy distribution and/or energy differences are compared with criteria, obtained by means of a mathematical reference model, for determining deviations in glass distributions and causes leading to thermal stresses in the product.

2. An analytical system according to claim 1, **characterized in that** said criteria obtained by means of a mathematical reference model depend on the associated location on or within the material of the product for which the energy differences and/or energy distribution are determined.

3. An analytical system according to any of the preceding claims, **characterized in that** said sensor system (24) comprises at least a detector element (26) with infrared-sensitive pixels arranged in rows or rows and columns.

4. An analytical system according to claim 3, **characterized in that** said digital processor (30) determines differences between the energy content of different pixels for obtaining said energy differences between different parts of the product (4).

5. An analytical system according to claim 4, **characterized in that** said energy differences are determined with coordinates reproducing the associated pixels of the detector element and representing a part of the product.

6. An analytical system according to claim 1, **characterized in that** said digital processor (30) determines the glass distribution of the product (4) by means of the energy distribution over the product (4).

7. An analytical system according to claim 1, **characterized in that** said digital processor (30) determines thermal stresses in the product by means of the energy differences and compares them with the criteria for obtaining information about a deviation in the product.

8. An analytical system according to any of the preceding claims, **characterized in that** said system (1) is further provided with an optical sensor system (50) connected with the digital processor (30), by means of which, in use, an image of products (4) is taken, said digital processor (30) comparing the information obtained from said image with the information obtained by the infrared-sensitive sensor (24) system for the same products (4), and adjusting or readjusting, by means of said comparison, criteria for analyzing the glass distribution over the product (4) or detecting deviations in the product (4).

9. An analytical system according to any of the preceding claims, **characterized in that** on the basis of the information obtained about deviations in a product (4) or a series of products (4), in use, control signals are generated by the analytical system (1) for regulating process parameters of the production process.

10. An analytical system according to claim 9, **characterized in that** said control signals are generated when the parameters obtained trendwise reproduce defects or a deviation of the desired quality.

11. An apparatus (2) for producing packaging glass products, partly by heating, provided with an analytical system (1) according to any of the preceding claims, in which a glass material (12) to be shaped into the packaging glass product is passed along a production stage comprising a first section (18) in which the glass material is molten, a second section (20), following the first section, in which the product is shaped from the glass material, and a third section (22), following the second section (20) , in which the glass material shaped into a product (4) is cooled, said infrared-sensitive sensor system (1) detecting products (4) at at least a point in the second section in which the products shaped are not yet cooled.

## Patentansprüche

1. Analysesystem (1) zum Analysieren, Überwachen, Diagnostizieren und/oder Steuern eines Verfahrens zur Herstellung von Glasverpackungsprodukten (4), wobei des Analysesystem (1) mit einem infrarotempfindlichen Sensorsystem (24) und einem damit verbundenen Digitalprozessor (30) versehen ist, wobei das infrarotempfindliche Sensorsystem (24) Infrarotstrahlung erkennt, die von warmen Produkten in dem Bereich unmittelbar nach dem Glasformvorgang abgestrahlt wird, und wobei der Digitalprozessor (30) die Energieverteilung in dem Material des geformten Produkts und Energiedifferenzen zwischen verschiedenen Teilen des geformten Produkts mittels Informationen über die Produkte ermittelt, die mit dem infrarotempfindlichen Sensorsystem (24) erhalten wurden, wobei die Energieverteilung und/oder Energiedifferenzen mit Kriterien verglichen werden, die mittels eines mathematischen Referenzmodells erhalten wurden, um Abweichungen in der Glasverteilung und Ursachen, die zu thermischen Belastungen im Produktführen, zu ermitteln.

2. Analysesystem nach Anspruch 1, **dadurch gekennzeichnet, daß** die durch ein mathematisches Referenzmodell erhaltenen Kriterien von der damit verbundenen Stelle auf oder in dem Material des Produkts abhängen, für welche die Energiedifferenzen und/oder die Energieverteilung ermittelt werden.

3. Analysesystem nach einem der vorhergehenden Ansprüche, **dadurch gekennzeichnet, daß** das Sensorsystem (24) wenigstens ein Detektorelement (26) aufweist, das in Reihen oder in Reihen und Spalten angeordnete infrarotempfindliche Pixel aufweist.

4. Analysesystem nach Anspruch 3, **dadurch gekennzeichnet, daß** der-Digitalprozessor (30) Differenzen zwischen dem Energiegehalt verschiedener Pixel ermittelt, um die Energiedifferenzen zwischen verschiedenen Teilen des Produkts (4) zu erhalten.

5. Analysesystem nach Anspruch 4, **dadurch gekennzeichnet, daß** die Energiedifferenzen mit Koordinaten bestimmt werden, welche die zugehörigen Pixel des Detektorelements reproduzieren und einen Teil des Produkts wiedergeben.

6. Analysesystem nach Anspruch 1, **dadurch gekennzeichnet, daß** der Digitalprozessor (30) die Glasverteilung des Produkts (4) durch die Energieverteilung im Produkt (4) bestimmt.

7. Analysesystem nach Anspruch 1, **dadurch gekennzeichnet, daß** der Digitalprozessor (30) thermische Belastungen im Produkt durch die Energiedifferenzen bestimmt und diese mit den Kriterien vergleicht, um Informationen über eine Abweichung im Produkt zu erhalten.

8. Analysesystem nach einem der vorhergehenden Ansprüche, **dadurch gekennzeichnet, daß** das System (1) ferner mit einem optischen Sensorsystem (50) versehen ist, das mit dem Digitalprozessor (30) verbunden ist, wobei mit diesem im Gebrauch ein Bild vorn Produkten (4) aufgenommen wird, wobei der Digitalprozessor (30) die aus dem Bild erhaltenen Informationen mit den durch das infrarotempfindliche Sensorsystem (24) für die selben Produkte (4) erhaltenen Informationen vergleicht und unter Verwendung des Vergleichs Kriterien für das Analysieren der Glasverteilung im Produkt (4) oder das Erkennen von Abweichungen im Produkt (4) einstellt oder neu einstellt.

9. Analysesystem nach einem der vorhergehenden Ansprüche, **dadurch gekennzeichnet, daß** auf der Basis der Informationen, die über Abweichungen in einem Produkt (4) oder einer Reihe von Produkten (4) erhalten wurden, im Gebrauch vom Analysesystem (1) Steuersignale zum Regeln von Verfahrensparametern des Herstellungsprozesses erzeugt werden.

10. Analysesystem nach Anspruch 9, **dadurch gekennzeichnet, daß** die Steuersignale erzeugt werden, wenn die erhaltenen Parameter einen Trend zur Wiederholung von Fehlern oder einer Abweichung von der gewünschten Qualität zeigen.

11. Vorrichtung (2) zum Herstellen von Glasverpackungsprodukten, teilweise durch Erwärmen, die mit einem Analysesystem (1) nach einem der vorhergehenden Ansprüche versehen ist, bei der ein zum Glasverpakkungsprodukt zu formendes Glasmaterial (12) durch eine Produktionsstufe geleitet, die einen ersten Abschnitt (18), in dem das Glasmaterial geschmolzen wird, einen dem ersten Abschnitt folgenden zweiten Abschnitt (20), in dem das Produkt aus dem Glasmaterial geformt wird, und einen dem zweiten Abschnitt (20) folgenden dritten Abschnitt (22), in dem das zu einem Produkt (4) geformte Glasmaterial abgekühlt wird, wobei das infrarotempfindliche Sensorsystem (1) Produkte (4) an wenigstens einer Stelle im zweiten Abschnitt erkennt, in dem die geformten Produkte noch nicht abgekühlt sind.

## Revendications

1. Système analytique (1) permettant d'effectuer une analyse, un contrôle, un diagnostic et, ou bien, une régulation sur un processus de fabrication de verres, ou produits en verre, pour emballages (4), ledit système analytique (1) étant doté d'un système capteur (24) sensible aux infrarouges et d'un dispositif de traitement numérique (30) connecté à celui-ci, ledit système capteur (24) sensible aux infrarouges détectant le rayonnement infrarouge émis par des produits chauds dans la section venant directement après le processus de formage du verre, et ledit dispositif de traitement numérique (30) déterminant la distribution énergétique dans le matériau du produit formé et les différences d'énergie entre parties différentes du produit formé au moyen d'informations venant des produits obtenus à l'aide dudit système capteur sensible aux infrarouges, où ladite distribution d'énergie et, ou bien, lesdites différences d'énergie sont comparées avec des critères, obtenus par l'intermédiaire d'un modèle de référence mathématique, afin de déterminer les écarts existant dans la distribution du verre et les causes conduisant à la présence de contraintes thermiques dans le produit.

2. Système analytique selon la revendication 1, **caractérisé en ce que** lesdits critères obtenus par l'intermédiaire d'un modèle de référence mathématique dépendent de la position associée sur le matériau ou à l'intérieur du matériau du produit pour lequel les différences d'énergie et, ou bien, la distribution d'énergie sont déterminées.

3. Système analytique selon l'une quelconque des revendications précédentes, **caractérisé en ce que** ledit système capteur (24) comprend au moins un élément détecteur (26) ayant des pixels sensibles aux infrarouges disposés en rangées ou en rangées et en colonne.

4. Système analytique selon la revendication 3, **caractérisé en ce que** ledit dispositif de traitement numérique (30) détermine des différences entre le contenu énergétique de différents pixels en vue d'obtenir lesdites différences d'énergie entre des parties différentes du produit (4).

5. Système analytique selon la revendication 4, **caractérisé en ce que** lesdites différences d'énergie sont déterminées avec des coordonnées reproduisant les pixels associés de l'élément détecteur et représentant une partie du produit.

6. Système analytique selon la revendication 1, **caractérisé en ce que** ledit dispositif de traitement numérique (30) détermine la distribution du verre dans le produit (4) par l'intermédiaire de la distribution d'énergie sur le produit (4).

7. Système analytique selon la revendication 1, **caractérisé en ce que** ledit dispositif de traitement numérique (30) détermine les contraintes thermiques présentes dans le produit par l'intermédiaire des différences d'énergie et les compare avec les critères afin d'obtenir des informations relatives à un écart présent dans le produit.

8. Système analytique selon l'une quelconque des revendications précédentes, **caractérisé en ce que** ledit système (1) est en outre doté d'un système capteur optique (50) connecté au dispositif de traitement numérique (30), par l'intermédiaire duquel, en utilisation, est prise une image de produits (4), ledit dispositif de traitement numérique (30) comparant les informations obtenues de la part de ladite image avec les informations obtenues par le système capteur (24) sensible aux infrarouges pour les mêmes produits (4), et ajustant, ou réajustant, via ladite comparaison, les critères d'analyse de la distribution du verre sur le produit (4) ou de détection d'écarts dans le produit (4).

9. Système analytique selon l'une quelconque des revendications précédentes, **caractérisé en ce que**, sur la base des informations obtenues sur des écarts existant dans un produit (4) ou une série de produits (4), en utilisation, des signaux de commande sont produits par le système analytique (1) afin de réguler des paramètres de traitement du processus de production.

10. Système analytique selon la revendication 9, **caractérisé en ce que** lesdits signaux de commande sont produits lorsque les paramètres obtenus reproduisent de manière tendantielle des défauts ou un écart par rapport à la qualité voulue.

11. Appareil (2) destiné à produire des verres, ou produits en verre, pour emballages, en partie par chauffage, qui est doté d'un système analytique (1) tel que défini dans l'une quelconque des revendications précédentes, où un matériau à base de verre (12) devant être formé en un produit d'emballage en verre passe dans un étage de production comprenant une première section (18) dans laquelle on fait fondre le matériau de verre, une deuxième section (20), faisant suite à la première section, dans laquelle on donne sa forme au produit à partir du matériau en verre, et une troisième section (22), faisant suite à la deuxième section (20), dans laquelle on refroidit le matériau en verre formé en un produit (4), ledit système capteur (1) sensible aux infrarouges détectant des produits (4) en au moins un point dans la deuxième section, dans laquelle les produits formés ne sont pas encore refroidis.
